# EUROPÄISCHE PATENTSCHRIFT

(11) **EP 1 107 699 B1**
(45) Veröffentlichungstag und Bekanntmachung des Hinweises auf die Patenterteilung: **05.11.2003**
(21) Anmeldenummer: 98938576.0
(22) Anmeldetag: 25.08.1998
(51) Int. Cl.: A61B 17/00

(54) **OSTEOSYNTHETISCHE FIXATIONSVORRICHTUNG**
OSTEOSYNTHETIC FASTENING DEVICE
DISPOSITIF DE FIXATION OSTEOSYNTHETIQUE

(43) Veröffentlichungstag der Anmeldung: 20.06.2001
(73) Patentinhaber: Medartis AG, 4051 Basel (CH)
(72) Erfinder: JOOS, Ulrich, D-48147 Münster (DE)
(74) Vertreter: Heinen, Detlef
(86) Internationale Anmeldenummer: CH9800366
(87) Internationale Veröffentlichungsnummer: WO98044849

(56) Entgegenhaltungen:
- WO-A-97/01991
- DE-A- 2 340 880
- DE-A- 4 018 273
- DE-U- 9 016 507

## Beschreibung

Die vorliegende Erfindung bezieht sich auf eine Vorrichtung zur osteosynthetischen Fixation von Knochenfragmenten, insbesondere von Fragmenten eines Kieferknochens. Derartige Vorrichtungen werden intraoperativ eingesetzt, um voneinander separierte Knochenfragmente zusammengefügt zu fixieren. Dies kann entweder nach Unfällen, wo ein Knochen in Knochenfragmente zerbrochen ist, innerhalb einer Osteosynthese erforderlich sein oder innerhalb einer orthognatischen bzw. maxillofacialen Behandlung zur chirurgischen Regelung abnormer Stellungen nach einer Osteotomie und anschliessender Positionskorrektur der Knochenfragmente. Eine solche Vorrichtung überbrückt und fixiert vorrangig zwei Knochenfragmente miteinander, wobei je eine Partie der Vorrichtung mit einem Knochenfragment lösbar verbunden ist. Zur Herstellung der Verbindung zwischen der temporär eingesetzten Vorrichtung und den Knochenfragmenten befinden sich in der Knochenplatte - diese stellt das wesentliche Bauelement der Vorrichtung dar -, Durchgangslöcher in denen Befestigungselemente, zumeist Knochenschrauben, stecken, welche in die Knochenfragmente eingreifen. Bei den hiesigen Vorrichtungen kommt es darauf an, dass die Knochenfragmente zueinander positionsgerecht und stabil fixiert werden.

### Stand der Technik

In der DE-C-23 40 880 wird eine massive Kompressionsplatte zur Behandlung von Kieferbrüchen offenbart, die die Bruchstelle am Kieferknochen überbrückend auf beide zusammenzufügenden Knochenkompartimente aufgeschraubt wird. In beiden Hälften der Kompressionsplatte sind je zwei zur Plattenmitte und zur Bruchstelle hin orientierte Langlöcher vorhanden. Die Langlöcher besitzen auf der dem Kieferknochen abgewendeten Seite eine Ansenkung mit einem als schiefe Ebene ausgebildeten Schraubensitz. Zumindest je ein Langloch pro Hälfte ist schräg zur Plattenmitte ausgerichtet. Auf der dem Kieferknochen zugewandten Seite besitzt die Knochenplatte mittig einen vorspringenden gekerbten Steg. Durch die Anordnung der Langlöcher und die schiefen Schraubensitze werden beim Anziehen der eingesetzten Knochenschrauben die Knochenkompartimente zur Bruchstelle hin komprimiert. Diese Platte ist durch ihre Steifheit kaum an die vorhandene Knochengeometrie anpassbar. Das simple Lochraster erlaubt wenig Variabilität für die Befestigung an den Knochenkompartimenten und ein vorheriges provisorisches Fixieren vor der definitiven Positionierung ist nun in sehr engen Bewegungsspielräumen möglich.

Eine weiter entwickelte Knochenplatte zur Fixation von Knochenkompartimenten ist Gegenstand der WO-A-97 01991. Die dort beschriebene Knochenplatte hat an ihren beiden Aussenseiten zumindest je ein durchgehendes Schraubenloch, zwischen denen sich ein erstes axiales Langloch befindet, das an den beiden Längsflanken von sich gegenüber liegenden Stegen begrenzt wird. Ferner ist ein klammerförmiger Feststeller zum Übergreifen der Knochenplatte in Querrichtung - d.h. über beide Stege mit dem dazwischen liegenden ersten Langloch - vorgesehen. Der Feststeller weist ein zweites Langloch auf, das sich quer zur Längsachse der Knochenplatte und somit auch quer zum ersten Langloch erstreckt. Die Längenausdehnung des zweiten Langlochs entspricht der Breite des ersten Langlochs. Der Knochenplatte zugewandt besitzt der Feststeller äusserlich zwei abgewinkelte Führungsnasen, die im aufgesetzten Zustand beide Stege unter- bzw. übergreifen, so dass der Feststeller wie auf einer Doppelschiene longitudinal auf der Knochenplatte verschiebbar ist.

In der Anwendung schraubt man eine Aussenseite der Knochenplatte an einem ersten Knochenkompartiment fest. Dann wird der Feststeller im Bereich des ersten Langlochs temporär auf die Knochenplatte aufgesetzt und mittels einer in das zweite Knochenkompartiment eingedrehten Feststellschraube nach dem Positionieren des beweglichen Knochenkompartiments fixiert. Das Positionieren geschieht durch longitudinale Relatiwerschiebung zwischen der Knochenplatte und dem Feststeller innerhalb des Spielraums des ersten Langlochs sowie durch transversale Relativverschiebung zwischen der Feststellschraube und Knochenplatte samt Feststeller innerhalb des Spielraums des zweiten Langlochs. Wurden Knochenplatte sowie Feststeller soweit fixiert und die Knochenkompartimente zueinander ausgerichtet, verschraubt man die verbleibende Aussenseite der Knochenplatte mit dem zweiten Knochenkompartiment. Hiernach wird die Feststellschraube gelöst und auch der Feststeller entfernt.

Mit der vorbeschriebenen Knochenplatte wurde gegenüber dem älteren Stand der Technik eine markante Verbesserung erzielt, da sie durch deren Biegsamkeit leichter und genauer an die jeweilige Knochengeometrie anpassbar ist. Überdies ermöglicht der Feststeller eine intraoperative zunächst annähernde und dann präzise Ausrichtung der beiden miteinander zusammenzufügenden Knochenkompartimente. Es verbleiben jedoch folgende Unvollkommenheiten:
a) Der Feststeller und die zugehörige Feststellschraube sind zwei relativ kleine separate Einzelteile, deren Applikation entsprechend heikel ist.
b) Die Knochenplatte besitzt eine fixe, nicht der jeweiligen Situation anpassbare Länge. Das erste longitudinale Langloch, welches sich in der Knochenplatte befindet, ist allseits geschlossen und liegt zwischen den Schraubenlöchern, wodurch die Variabilität in der Längsbeweglichkeit limitiert wird.
c) Die Knochenplatte ist nur im Bereich einer Steifigkeit an die zusammenzufügenden Knochenkompartimente anschraubbar; von Vorteil wäre auch hier eine grössere Variabilität nutzen zu können.

### Aufgabe der Erfindung

Angesichts der aufgezeigten Unvollkommenheiten der bis dato bekannten Vorrichtungen zur osteosynthetischen Fixation von Knochenfragmenten mit einem temporär aufgesetzten Feststeller liegt der Erfindung die Aufgabe zugrunde, die zur Vorrichtung gehörige Knochenptatte derart zu gestalten, dass deren Anpassbarkeit an die jeweils vorgefundene Knochensituation durch leichtere Verformbarkeit, vereinfachte Längenwahl und grössere Variabilität bei der Einstellung lokal differenzierter Steifigkeiten verbessert wird. Ausserdem soll ein möglichst grosser Bewegungsspielraum für die mit einem Feststeller vorfixierte Knochenplatte zur Verfügung stehen. Ferner gilt es, das Handling mit dem bisher zweiteiligen Feststeller und der Knochenplatte während der Operation zu vereinfachen. Schliesslich muss sich die gesamte Vorrichtung auf effiziente Weise in Serie fertigen lassen.

### Übersicht über die Erfindung

In einer ersten Ausführung der Fixationsvorrichtung weist die Knochenplatte einen von Plattenstreben begrenzten longitudinalen Führungsschlitz auf, der einseitig offen oder beidseits geschlossen ist. Ein temporär auf die Knochenplatte aufzusetzender Feststeller besitzt eine die Knochenplatte partiell übergreifende Scheibe, in der drehbar oder feststehend eine Feststellschraube steckt, wobei letztere zum Einschrauben in ein Knochenkompartiment durch den Führungsschlitz vorgesehen ist. Feststeller und Knochenplatte mit den jeweils angeschraubten Knochenkompartimenten sind innerhalb der Länge und der Breite des Führungsschlitzes relativ zueinander verschiebbar. An der Knochenplatte sind zumindest an einem Ende mehrere Schraubenlöcher vorgesehen. Mit Vorteil hat die Knochenplatte an einem Ende einen Gliederbereich mit abtrennbaren Gliedern, um die jeweils erforderliche Länge zuschneiden zu können. Mit mehreren Möglichkeiten des Festschraubens der Knochenplatte erzielt man eine bessere Anpassbarkeit an die jeweilige Aufgabe und eine verbesserte Steifheit über die Kante der Knochenplatte betrachtet, während deren Elastizität über die Fläche durch geringere Materialstärke gesteigert werden kann.

Vorzugsweise ist die auf der Feststellschraube des Feststellers steckende Scheibe gegen Herunterfallen gesichert. Hierdurch hat man quasi einen einteiligen Feststeller mit vereinfachtem Handling. Zur zusätzlichen Versteifung der aufgeschraubten Knochenplatte und/oder zur einseitigen Verschraubung der Knochenplatte ist eine Fixierplatte vorgesehen, die auf die anderenends durch Schraubenlöcher mittels Knochenschrauben verschraubte Knochenplatte aufsetzbar ist.

In einer zweiten Ausführung besitzt der auf die Knochenplatte aufsetzbare Feststeller eine die Knochenplatte partiell übergreifende Platte, welche seitlich, ausserhalb der Knochenplatte mit dem darunter befindlichen Knochenkompartiment verschraubbar ist.

### Kurzbeschreibung der beigefügten Zeichnungen

Es zeigen:
- Figur 1A -: eine einseitig lateral offene Knochenplatte mit horizontalem Verbindungssteg, je einem Gliederbereich an zwei zueinander parallelen, frei endenden Plattenstreben und einem zwischen Verbindungssteg und den Gliederbereichen sich erstreckendem Führungsschlitz in der Draufsicht sowie einen zugeordneten Feststeller in Drauf- und Seitenansicht;
- Figur 1B -: die Knochenplatte gemäss Figur 1A mit im Führungsschlitz eingesetztem Feststeller, eingesetzten Knochenschrauben und zwei symmetrisch abgetrennten Lochgliedern;
- Figur 1C -: die Knochenplatte gemäss Figur 1A vergrössert;
- Figur 1D -: den Feststeller gemäss Figur 1A vergrössert als Teilschnitt in der Seitenansicht;
- Figur 1E -: den Feststeller gemäss Figur 1A vergrössert in der Draufsicht;
- Figur 2A -: die Darstellung gemäss Figur 1A mit einem sich zwischen den Gliederbereichen fortsetzenden, tailliert austretendem Führungsschlitz;
- Figur 2B -: die Knochenplatte gemäss Figur 2A mit im Führungsschlitz, zwischen den Gliederbereichen, eingesetztem Feststeller und zwei symmetrisch abgetrennten Lochgliedern;
- Figur 3A -: die Darstellung gemäss Figur 2A mit einem zwischen den Gliederbereichen glatt austretendem Führungsschlitz;
- Figur 3B -: die Knochenplatte gemäss Figur 3A mit im Führungsschlitz, zwischen den Gliederbereichen, eingesetztem Feststeller und zwei symmetrisch abgetrennten Lochgliedern;

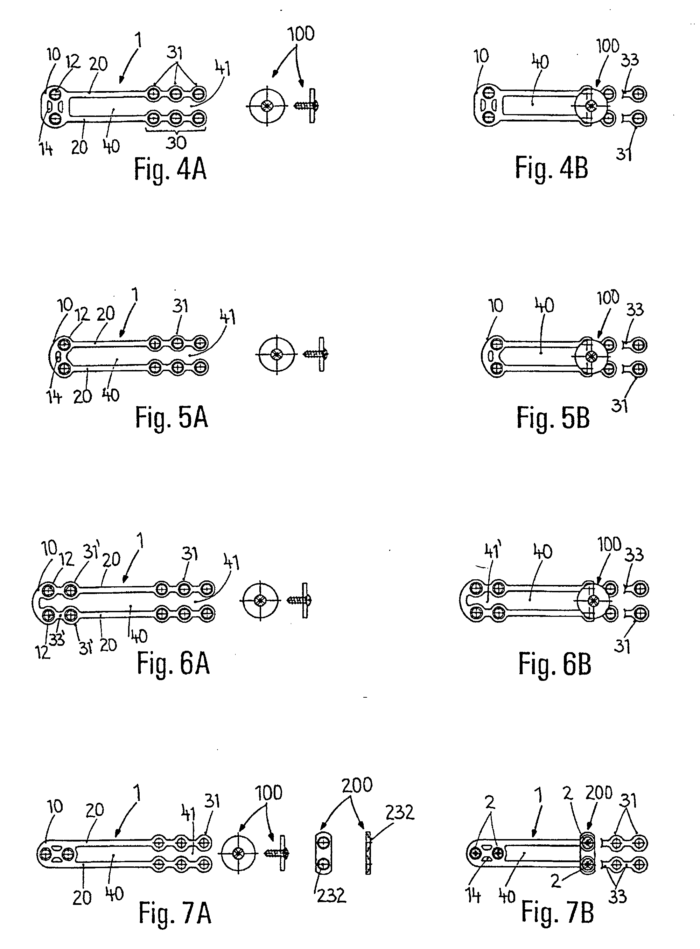

- Figur 4A -: die Darstellung gemäss Figur 2A mit vertikalem Verbindungssteg;
- Figur 4B -: die Knochenplatte gemäss Figur 4A mit im Führungsschlitz, zwischen den Gliederbereichen, eingesetztem Feststeller und zwei symmetrisch abgetrennten Lochgliedern;
- Figur 5A -: die Darstellung gemäss Figur 2A mit nierenförmigem Verbindungssteg;
- Figur 5B -: die Knochenplatte gemäss Figur 5A mit im Führungsschlitz, zwischen den Gliederbereichen, eingesetztem Feststeller und zwei symmetrisch abgetrennten Lochgliedern;
- Figur 6A -: die Darstellung gemäss Figur 5A mit nierenförmigem Verbindungssteg hinter einem zusätzlichen Paar von Lochgliedern;
- Figur 6B -: die Knochenplatte gemäss Figur 6A mit im Führungsschlitz, zwischen den Gliederbereichen, eingesetztem Feststeller und zwei symmetrisch abgetrennten Lochgliedern;
- Figur 7A -: die Darstellung gemäss Figur 1A mit einer zusätzlichen streifenförmigen Fixierplatte in Drauf- und Seitenansicht;
- Figur 7B -: die Knochenplatte mit aufgeschraubter Fixierplatte gemäss Figur 7A, eingesetzten Knochenschrauben und vier symmetrisch abgetrennten Lochgliedern;
- Figur 7C -: die Knochenplatte gemäss den Figuren 1A und 7A vergrössert;

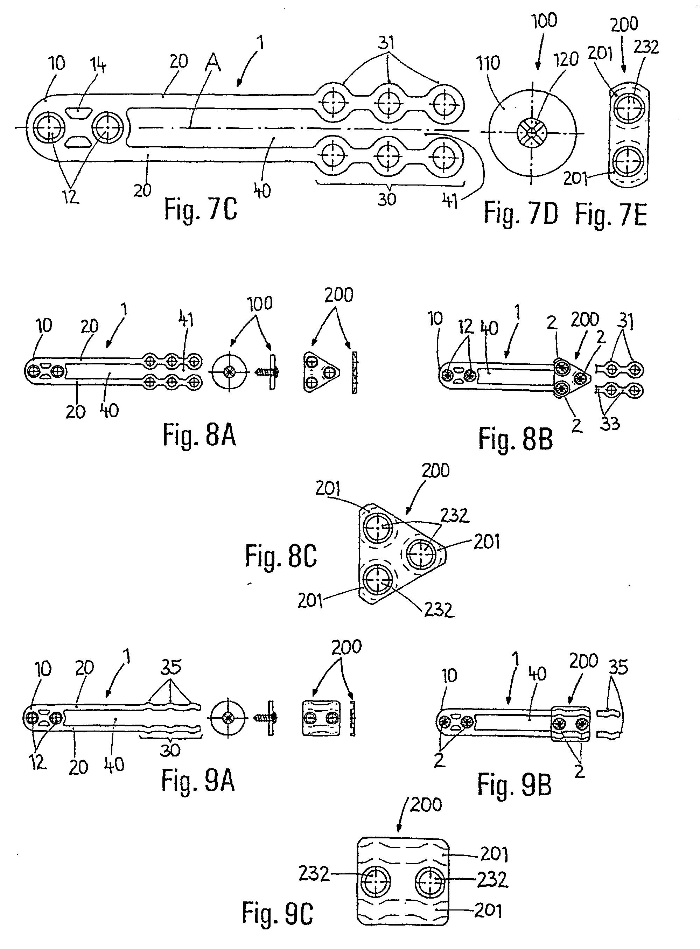

- Figur 7D -: den Feststeller gemäss den Figuren 1A und 7A vergrössert in der Draufsicht;
- Figur 7E -: die Fixierplatte gemäss Figur 7A vergrössert in der Draufsicht;
- Figur 8A -: die Darstellung gemäss Figur 7A mit einer dreieckförmigen Fixierplatte;
- Figur 8B -: die Knochenplatte gemäss Figur 7B mit aufgeschraubter Fixierplatte gemäss Figur 8A,
- Figur 8C -: die Fixierplatte gemäss den Figuren 8A und 8B vergrössert in der Draufsicht;
- Figur 9A -: eine einseitig lateral offene Knochenplatte mit horizontalem Verbindungssteg, sich vom Verbindungssteg erstreckendem Führungsschlitz und wellig, frei endenden Plattenstreben in der Draufsicht sowie zugeordnet einem Feststeller und einer zusätzlichen, etwa quadratischen Fixierplatte jeweils in Drauf- und Seitenansicht;
- Figur 9B -: die Knochenplatte gemäss Figur 9A mit aufgeschraubter Fixierplatte gemäss Figur 9A, eingesetzten Knochenschrauben und zwei von den Plattenstreben symmetrisch abgetrennten Strebengliedern;
- Figur 9C -: die Fixierplatte gemäss den Figuren 9A und 9B vergrössert in der Draufsicht;
- Figur 10A -: eine einseitig lateral offene Knochenplatte mit horizontalem Verbindungssteg, sich vom Verbindungssteg erstreckendem Führungsschlitz und gerade, frei endenden Plattenstreben in der Draufsicht sowie zugeordnet einer zusätzlichen, rundlichen Fixierplatte mit einem Mittelsteg in Drauf- und Seitenansicht;
- Figur 10B -: die Knochenplatte gemäss Figur 10A mit aufgesetzter Fixierplatte;
- Figur 10C -: die Fixierplatte gemäss den Figuren 10A und 10B vergrössert in der Draufsicht;
- Figur 11A -: die Darstellung gemäss Figur 10A ohne Mittelsteg an der rundlichen Fixierplatte;
- Figur 11B -: die Knochenplatte gemäss Figur 11A mit aufgesetzter Fixierplatte;
- Figur 11C -: die Fixierplatte gemäss den Figuren 11A und 11B vergrössert in der Draufsicht;
- Figur 12A -: eine beidseits lateral geschlossene Knochenplatte mit nierenförmigen Verbindungsstegen, denen je ein Paar von Lochgliedern vorgelagert ist und einem zwischen den Lochgliedern sich erstreckenden Führungsschlitz sowie einen zugeordneten Feststeller jeweils in Draufsicht;
- Figur 12B -: die Knochenplatte gemäss Figur 12A mit im Führungsschlitz eingesetztem Feststeller;
- Figur 12C -: die Knochenplatte gemäss Figur 12A vergrössert in Draufsicht;
- Figur 12D -: die Knochenplatte gemäss Figur 12C in Frontansicht;
- Figur 13A-: die Darstellung gemäss Figur 12A mit einem rechts zusätzlich vorhandenen Paar von Lochgliedern und einem sich zwischen den Lochgliedern fortsetzenden Führungsschlitz;
- Figur 13B - die: Knochenplatte gemäss Figur 13A mit im Führungsschlitz, zwischen den Lochgliedern, eingesetztem Feststeller und einem symmetrisch abgetrennten Verbindungssteg;
- Figur 14A -: die Darstellung gemäss Figur 12A ohne den Verbindungsstegen vorgelagerten Lochgliedern;
- Figur 14B-: die Knochenplatte gemäss Figur 14A mit im Führungsschlitz eingesetztem Feststeller;
- Figur 15A -: eine beidseits lateral geschlossene Knochenplatte mit in Form eines Parallelogramms angeordneten Verbindungsstegen und einem zwischen den Verbindungsstegen sich erstreckenden Führungsschlitz sowie einen zugeordneten Feststeller jeweils in Draufsicht;
- Figur 15B -: die Knochenplatte gemäss Figur 15A mit im Führungsschlitz eingesetztem Feststeller.
- Figur 16A - 16D: zeigen keine erfindungsgemäße Vorrichtung.

Ferner zeigen :
- Figur 17A -: den Feststeller gemäss Figur 1D mit einer auf dem Schraubenschaft drehbaren, nicht lösbaren, planförmigen Scheibe;
- Figur 17B -: den Feststeller gemäss Figur 17A in der Draufsicht;
- Figur 18A-: die Darstellung gemäss Figur 17A mit einer Scheibe, die auf ihrer Unterseite Arretiemasen aufweist;
- Figur 18B -: den Feststeller gemäss Figur 18A in der Draufsicht;
- Figur 19A -: die Darstellung gemäss Figur 17A mit einer gewölbten Scheibe;
- Figur 19B -: den Feststeller gemäss Figur 19A in der Draufsicht;
- Figur 20A -: einen einteiligen Feststeller mit einer auf dem Schraubenschaft fest sitzenden, planförmigen Scheibe;
- Figur 20B -: den Feststeller gemäss Figur 20A in der Draufsicht;
- Figur 21A -: einen einteiligen Feststeller mit einer auf dem Schraubenschaft fest sitzenden, gewölbten Scheibe; und
- Figur 21 B-: den Feststeller gemäss Figur 21A in der Draufsicht.

### Ausführungsbeispiele

Mit Bezug auf die beiliegenden Zeichnungen erfolgt nachstehend die detaillierte Beschreibung von Ausführungsbeispielen zur erfindungsgemässen Fixationsvorrichtung.

Für die gesamte weitere Beschreibung gilt folgende Festlegung. Sind in einer Figur zum Zweck zeichnerischer Eindeutigkeit Bezugsziffern enthalten, aber im unmittelbar zugehörigen Beschreibungstext nicht erläutert, so wird auf deren Erwähnung in vorangehenden oder nachfolgenden Figurenbeschreibungen Bezug genommen. Im Interesse der Übersichtlichkeit wird auf die wiederholte Bezeichnung von Bauteilen in weiteren Figuren zumeist verzichtet, sofern zeichnerisch eindeutig erkennbar ist, dass es sich um "wiederkehrende" Bauteile handelt.

### Figuren 1A und 1C

Die Fixationsvorrichtung besteht aus einer Knochenplatte **1** - hier einseitig lateral offen und mit horizontalem Verbindungssteg **10** - sowie einem Feststeller **100**. Vom Verbindungssteg **10** verlaufen zwei zueinander parallel beabstandete Plattenstreben **20**, an deren Enden je ein Gliederbereich **30** mit je drei Lochgliedern **31** vorgesehen sind, die sich paarweise eng gegenüber stehen. Zwischen dem Verbindungssteg **10** und dem Gliederbereich **30** erstreckt sich ein Führungsschlitz **40**, der im Gliederbereich **30** zu einem dreifach taillierten Spalt **41** verengt und diametral gegenüber dem Verbindungssteg **10** austritt. Die Lochglieder **31** sind augenförmig und haben jeweils ein zentrisches Schraubenloch **32** sowie einen Übergangssteg **33** zum benachbarten Lochglied **31**. Folglich besitzt das mittlere Paar von Lochgliedern **31** pro Lochglied **31** je zwei sich diametral gegenüber stehende Übergangsstege **33**, welche die Verbindung zum inneren und zum äusseren Paar von Lochgliedern **31** herstellen. An das innere Paar von Lochgliedem **31** schliessen sich gegenüber den Übergangsstegen **33** die beiden Plattenstreben **20** an, während das äussere Paar von Lochgliedern **31** nur Übergangsstege **33** aufweist, die dem mittleren Paar von Lochgliedern **31** zugewandt sind. Longitudinal durch die Knochenplatte **1** lässt sich eine theoretische Längsachse **A** legen, zu der die Knochenplatte **1** symmetrisch aufgebaut ist. Der Verbindungssteg **10** hat zwei auf der Längsachse **A** liegende Schraubenlöcher **12**, zwischen denen zwei Durchbrüche **14** in symmetrischen Abständen zur Längsachse **A** angeordnet sind. Die Durchbrüche **14** dienen dem Eingriff für ein an sich bekanntes Halteinstrument, um die Knochenplatte **1** während der Operation zum Einsatzort zu bringen.

Der Feststeller **100** besteht aus einer kreisrunden Scheibe **110** in der zentrisch eine Feststellschraube **120** steckt. Scheibe **110** und Feststellschraube **120** können zusammen ein Teil bilden oder sind so miteinander verbunden, dass die Feststellschraube **120** in der Scheibe **110** steckend drehbar ist, die Scheibe **110** sich aber nicht von der Feststellschraube **120** lösen kann.

### Figur 1B

Der Feststeller **100** ist dazu vorgesehen, auf die zuerst am ersten Knochenkompartiment angeschraubte Knochenplatte **1** aufgesetzt zu werden. Das einseitige Anschrauben mittels Knochenschrauben **2** könnte sowohl an den Gliederbereichen **30** als auch am Verbindungssteg **10** durch die Schraubenlöcher **12** erfolgen, im Regelfall wird man nach der letztgenannten Alternative verfahren. Nach einer Grobjustierung beider Knochenkompartimente setzt man den Feststeller **100** auf die Knochenplatte **1** auf und schraubt den Feststeller **100** mittels der Feststellschraube **120** am zweiten Knochenkompartiment zunächst lose an, so dass die Knochenplatte **1** unter dem Feststeller **100** für die Feinjustierung beider Knochenkompartimente zueinander noch beweglich bleibt. Die Feststellschraube **120** durchragt den Führungsschlitz **40**, greift in das darunter befindliche zweite Knochenkompartiment ein und die Scheibe **110** des Feststellers **100** überspannt den Abstand zwischen beiden Plattenstreben **20**. Zur Feinjustierung sind also noch Relativbewegungen zwischen beiden Knochenkompartmenten möglich, d.h. die Knochenplatte **1** mit dem daran fest angeschraubten Knochenkompartiment und das andere Knochenkompartiment mit dem aufgesetzten Feststeller **100** sind zueinander im Bereich des Bewegungsspielraums, welcher für den Feststeller **100** mit der Feststellschraube **120** innerhalb des Führungsschlitzes **40** verbleibt, beweglich.

Nach erfolgter Feinjustierung beider Knochenkompartimente zueinander, wird die Feststellschraube **120** angezogen, so dass die Scheibe **110** klemmend auf die beiden Plattenstreben **20** drückt und damit ist die eingerichtete Positionierung der beiden Knochenkompartimente fixiert. Jetzt bringt man die Knochenschrauben **2** auf der Seite des Verbindungssteges **10** in die Schraubenlöcher **12** ein. Wird nicht die gesamte Länge der Plattenstreben **20** mit den verlängernden Gliederbereichen **30** benötigt, so kann man überzählige Lochglieder **31** abtrennen. Um scharfe Kanten zu vermeiden, wird man dabei auch die anhängenden Übergangsstege **33** mit abtrennen. In welcher Länge man die Knochenplatte **1** einsetzt, welche Schraubenlöcher **32** bzw. **12** mit Knochenschrauben **2** belegt werden und ob anpassende Biegungen an der Knochenplatte **1** noch vorgenommen werden, hängt von der jeweiligen lokalen Knochengeometrie und der Zielstellung ab.

Sind beide Enden der Knochenplatte **1** mittels Knochenschrauben **2** an den beiden Knochenkompartimenten festgeschraubt, d.h. auf der Seite der Gliederbereiche **30** und auf der Seite des Verbindungssteges **10**, dann hat der Feststeller **100** seine temporäre Funktion erfüllt. Man dreht die Feststellschraube **120** aus dem darunter befindlichen Knochenkompartiment heraus und entfernt den Feststeller **100** von der Knochenplatte **1**.

### Figuren 1D und 1E

In der kreisrunden Scheibe **110** des Feststellers **100** befindet sich zentrisch eine durchgehende Bohrung **111** mit einer Ansenkung **112** auf der Scheibenoberseite **113**. Im hiesigen Ausführungsbeispiel hat die Bohrung **111** ein Innengewinde. In der Ansenkung **112** ist die untere Partie des Schraubenkopfes **122** der Feststellschraube **120** partiell eingebettet. Der Schraubenschaft **123** mit dem Aussengewinde **121** durchragt die Bohrung **111**, wobei Aussengewinde **121** und Innengewinde der Bohrung **111** miteinander in Eingriff stehen. Die Scheibe **110** kann dadurch nicht von der Feststellschraube **120** abfallen. Auf der Oberseite besitzt der Schraubenkopf **122** eine Eingriffskontur **124** - hier einen Kreuzschlitz - zum Ansetzen eines gebräuchlichen Eindrehwerkzeugs. Wie später beschrieben wird (s. Figuren 17A bis 21B), gibt es weitere Möglichkeiten zu verhindern, dass die Scheibe **110** von der Feststellschraube **120** abfällt.

### Figuren 2A und 2B

Diese Ausführung der Knochenplatte 1 unterscheidet sich dadurch, dass die Knochenplatte **1** insgesamt breiter ist, so dass die Plattenstreben **20** weiter auseinander liegen und ein breiterer Führungsschlitz **40** entsteht. Somit liegen auch die Gliederbereiche **30** mit den Lochgliedern **31** an beiden Plattenstreben **20** weiter auseinander, wodurch sich ein vergrösserter Spalt **41** ergibt. Der Führungsschlitz **40** ist quasi um den sich anschliessenden Spalt **41** verlängert. Dies erbringt insbesondere einen erweiterten longitudinalen Bewegungsspielraum bei der Feinjustierung, da der Feststeller **100** innerhalb eines verlängerten Führungsschlitzes **40**, also auch innerhalb der Gliederbereiche **30**, eingeschraubt werden kann. Die Feststellschraube **120** sitzt jetzt zwischen den Lochgliedern **31**.

### Figuren 3A und 3B

Einen verlängerten Führungsschlitz **40**, der sich ebenfalls zusätzlich über den Spalt **41** zwischen den Gliederbereichen **30** erstreckt, hat man hier erzeugt, indem die Lochglieder **31** mit den Schraubenlöchern **32** gänzlich nach aussen weisen, d.h. nicht in den Verlauf des Spalts **41** hineinragen, wodurch der Spalt **41** hin zu den Lochgliedern **31** glatte Kanten hat. Auch so eröffnet sich die Möglichkeit, den Feststeller **100** mit der Feststellschraube **120** nicht nur im Führungsschlitz **40**, sondern auch - wie dargestellt - in dessen Verlängerung, innerhalb des Spalts **41** zu positionieren.

### Figuren 4A und 4B

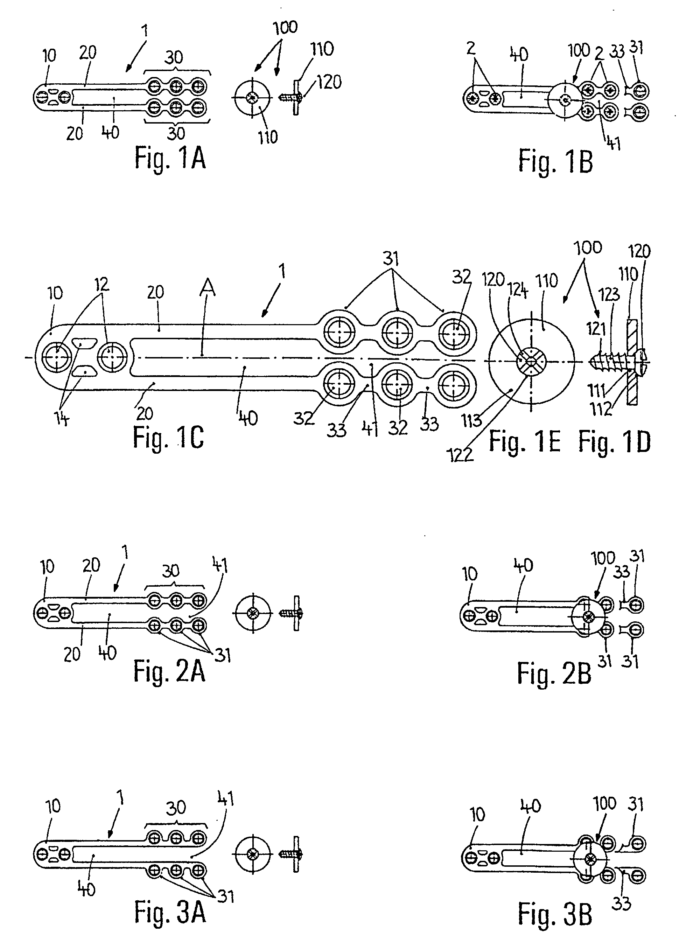

In Abweichung zur Figur 2A ist der Verbindungssteg **10** vertikal angeordnet. Die beiden Durchbrüche **14** liegen auf der Längsachse **A**, während die Schraubenlöcher **12** symmetrisch beabstandet zur Längsachse **A** angeordnet sind. Im gezeigten Ausführungsbeispiel liegen die Plattenstreben **20** mit den anschliessenden Gliederbereichen **30** soweit auseinander, dass sich erneut ein um den Spalt **41** verlängerter Führungsschlitz **40** ergibt. Der Feststeller **100** kann somit über die gesamte Länge des Führungsschlitzes **40** und anschliessendem Spalt **41** positioniert werden.

### Figuren 5A und 5B

Wie gezeigt, lässt sich der Verbindungssteg **10** auch nierenförmig gestalten. Die beiden darin befindlichen Schraubenlöcher **12** sind wiederum symmetrisch beabstandet zur Längsachse A angeordnet; auf der Längsachse A liegt ein Durchbruch **14**. Ansonsten besteht Identität zur vorherigen Ausführungsform.

### Figuren 6A und 6B

Bei dieser Gestaltungsvariante hat man an der Knochenplatte **1** den nierenförmigen Verbindungssteg **10** schmäler ausgebildet, auf Durchbrüche **14** gänzlich verzichtet und den Schraubenlöchern **12** im Verbindungssteg **10** ein Paar von Lochgliedern **31'** symmetrisch zur Längsachse **A** vorgelagert. Die zusätzlich angeordneten Lochglieder **31**' erweitern die Möglichkeiten der Befestigung der Knochenplatte **1** und damit auch die Einstellung verschiedener Steifigkeiten der Knochenplatte **1**. Einerseits schliesst jedes zusätzliche Lochglied **31'** an eine der Plattenstreben **20** an, andererseits besitzt jedes Lochglied **31'** einen Übergangssteg **33'**, der in der Flucht der Plattenstrebe **20** an den Verbindungssteg **10** anschliesst. Mit genügendem Abstand quer zur Längsachse **A** zwischen den Lochgliedern **31'**, so dass der Schraubenschaft **123** der Feststellschraube **120** den Zwischenraum zwischen dem Paar von Lochgliedern **31'** und eventuell auch den Zwischenraum zwischen den Schraubenlöchern **12** passieren kann, entsteht auch auf der Seite des Verbindungsstegs **10** ein Spalt **41'**, welcher den Führungsschlitz **40** in dieser Richtung bis an den Verbindungssteg **10** heran verlängert. Knochenplatte **1** und Feststeller **100** lassen sich nun relativ zueinander über die Strecke des Führungsschlitzes **40** mit den beidseitigen Verlängerungen durch die Spalte **41,41'** verschieben. Gezeigt ist die Positionierung des Feststellers **100** im Spalt **41** innerhalb der Gliederbereiche **30**, jedoch lässt sich die Knochenplatte **1** auch fixieren, wenn die Feststellschraube **120** im Führungsschlitz **40** oder im Spalt **41'** steht. Wie in allen vorherigen Darstellungen ist beispielhaft ein nicht benötigtes Paar von Lochgliedern **31** samt Übergangsstegen **33** von den Gliederbereichen **30** der Knochenplatte **1** abgetrennt.

### Figuren 7A und 7C bis 7E

Die Knochenplatte **1** und der Feststeller **100** entspricht der Gestaltung gemäss den Figuren 1A und 1D. Zur Erhöhung der Steifigkeit der aufgeschraubten Knochenplatte **1**, nach dem Entfernen des zuvor temporär eingesetzten Feststellers **100**, ist eine streifenförmige Fixierplatte **200** vorgesehen. Die Fixierplatte **200** besitzt Schraubenlöcher **232**, die kongruent zu einem Paar von beidseits der Längsachse **A** der Knochenplatte **1** angeordneten Schraubenlöchern **32** liegen. An der Unterseite der Fixierplatte **200**, welche der Knochenplatte **1** zugewandt ist, befinden sich um den Austritt der beiden Schraubenlöcher **232** Vertiefungen **201**, in denen das Paar von Lochgliedern **31** der Knochenplatte **1** zu liegen kommt.

### Figur 7B

Die Fixierplatte **200** kann man auf die festgeschraubte Knochenplatte **1** aufsetzen noch während der Feststeller **100** eingesetzt ist. Die aufgesetzte Fixierplatte **200** überbrückt unter sich die zwei beidseits der Längsachse **A** liegenden Lochglieder **31**, wobei die eingedrehten Knochenschrauben **2** mit ihren Köpfen auf der Oberseite der Fixierplatte **200** aufsitzen, während die Schäfte der Knochenschrauben **2** sowohl die Schraubenlöcher **232** in der Fixierplatte **200** als auch die Schraubenlöcher **32** in den Lochgliedem **31** der Knochenplatte **1** durchdringen und in das darunter befindliche Knochenkompartiment eingreifen. Wie vorbeschrieben, sind im verschraubten Zustand die Schraubenlöcher **12** im Verbindungssteg **10** ebenfalls mit Knochenschrauben **2** belegt, um diese Seite der Knochenplatte **1** mit dem anderen Knochenkompartiment zu verschrauben. Im hiesigen Beispiel waren zwei Paare von Lochgliedem **31** unnötig, die jeweils mit dem benachbarten Lochglied **31** zusammenhängend, samt Übergangsstegen **33**, abgetrennt sind.

### Figuren 8A bis 8C

Gegenüber der vorherigen Figurengruppe 7A bis 7E ist die Fixierplatte **200** jetzt gleichmässig dreieckförmig und enthält in der dritten Ecke, welche auf der Längsachse **A** liegt, zu den bei der streifenförmigen Fixierplatte **200** vorhandenen zwei Schraubenlöchern **232** ein weiteres Schraubenloch **232**. Um die Fixierplatte **200** in jeder der drei möglichen Drehstellungen passgerecht auf die Knochenplatte **1** aufsetzen zu können, befinden sich an der Unterseite der Fixierplatte **200**, am Austritt aller drei Schraubenlöcher **232**, Vertiefungen **201**. Im aufgesetzten Zustand liegt ein Paar von Lochgliedern **31** der Knochenplatte **1** mit den Schraubenlöchem **32** kongruent unter der Fixierplatte **200** mit den Schraubenlöchern **232**, die mittels Knochenschrauben **2** verschraubt sind. Das dritte, quasi freie Schraubenloch **232** der Fixierplatte **200** ist auch zum Einsetzen einer Knochenschraube **2** nutzbar, welche dann direkt in das darunter befindliche Knochenkompartiment eingreift.

### Figuren 9A bis 9C

Die Abwandlung dieser einseitig lateral offenen Knochenplatte **1** mit horizontalem Verbindungssteg **10** besteht darin, dass die am Verbindungssteg **10** entspringenden Plattenstreben **20** mit einem welligen Gliederbereich **30** enden, keine Lochglieder **31** aufweisen und der Führungsschlitz **40** sich damit vom Verbindungssteg **10** bis zum Austritt an den freien Enden der Plattenstreben **20** erstreckt. Am Verbindungssteg **10** und am Feststeller **100** gibt es keine Änderungen, hingegen ist die Fixierplatte **200** in Relation zu den abgewandelten Enden der Plattenstreben **20** modifiziert. Da man am freien Ende der Plattenstreben **20** hier auf Lochglieder **31** mit Schraubenlöchem **32** verzichtete, hat die Fixierplatte **200** neben der bisherigen Versteifungsfunktion hauptsächlich die Funktion, dieses Ende der Knochenplatte **1** mit dem darunter befindlichen Knochenkompartiment überhaupt verschrauben zu können.

Die freien Enden beider Plattenstreben **20** besitzen zur Längsachse **A** symmetrische, sich nach aussen wölbende und systematisch wiederholende Buckel, so dass sich an jeder Plattenstrebe **20** ein Gliederbereich **30** mit mehreren formgleichen, aneinander gereihten Strebengliedern **35** ergibt. Die Fixierplatte **200** ist für die hiesige Knochenplatte **1** etwa quadratisch gestaltet und weist zwei zueinander beabstandete, auf der Längsachse **A** liegende Schraubenlöcher **232** auf. An der Unterseite der Fixierplatte **200** befinden sich komplementär zur Form der Gliederbereiche **30** zwei ebenfalls symmetrisch zur Längsachse **A** liegende Vertiefungen **201**, die sich folglich in der Verlaufsrichtung der Gliederbereiche **30** erstrecken.

Im verschraubten Zustand liegt die Fixierplatte **200** über beiden Gliederbereichen **30**, wobei diese in den Vertiefungen **201** partiell eingebettet sind. Mittels Knochenschrauben **2**, welche die Schraubenlöcher **232** an der Fixierplatte **200** und die Schraubenlöcher **12** am Verbindungssteg **10** der Knochenplatte **1** durchdringen, ist letztere mit beiden Knochenkompartimenten verschraubt. Überstehende, nicht benötigte Strebenglieder **35** sind von den Gliederbereichen **30** abgeschnitten.

### Figuren 10A bis 10C

Hier sind die freien Enden der Plattenstreben **20** ohne Gliederbereich **30** einfach gerade ausgeführt, so dass sich vom horizontalen Verbindungssteg **10** der Führungsschlitz **40** gleichförmig bis zum Ende der Plattenstreben **20** erstreckt. Die rundliche Fixierplatte **200** besitzt zwei auf der Längsachse **A** liegende, zueinander beabstandete Schraubenlöcher **232**, zwischen denen symmetrisch zur Längsachse **A** zwei Durchbrüche **214** angeordnet sind. Die Durchbrüche **214** haben den gleichen Zweck, wie die Durchbrüche **14** in der Knochenplatte **1.** Kongruent zur Erstreckung der Plattenstreben **20** sind an der Unterseite der Fixierplatte **200**, parallel zur Längsachse **A**, zwei nutenförmige Vertiefungen **201** vorhanden. Zwischen den Vertiefungen **201**, im axialen Bereich der Schraubenlöcher **232**, verbleibt dadurch ein erhabener Mittelsteg **202**. Die Fixierplatte **200** könnte über die ganze Länge der Plattenstreben **20** auf diese aufgesetzt werden, realistisch - wegen der Verschraubung zweier Knochenkompartimente und der zu erreichenden Steifigkeit - ist jedoch eine Anordnung der Fixierplatte **200** nur nahe den Enden der Plattenstreben **20**. Im aufgesetzten Zustand sind die Plattenstreben **20** partiell in den Vertiefungen **201** eingebettet.

### Figuren 11A bis 11C

Der einzige Unterschied zur vorherigen Figurengruppe 10A bis 10C besteht darin, dass an der Unterseite der Fixierplatte **200** die Vertiefungen **201** nicht durch einen Mittelsteg **202** unterteilt sind, sondern sich die Vertiefung **201** durchgehend bis nahe an den oberen und unteren Rand der Fixierplatte **200** erstreckt.

### Figuren 12A bis 12D

Die Knochenplatte **1** ist beidseits lateral geschlossen und besitzt an beiden Aussenseiten nierenförmige Verbindungsstege **10**. Der linke Verbindungssteg **10** weist zwei zur Längsachse **A** symmetrisch beabstandete Schraubenlöcher **12** auf, denen mit Übergangsstegen **33'** verbunden, ein Paar von Lochgliedern **31'** vorgelagert ist. Rechte und linke Seite der Knochenplatte **1** sind identisch, also symmetrisch zu einer errichteten Vertikalachse **B**, die senkrecht zur Längsachse **A** steht. Um die eingeführte Systematik beizubehalten, wird auf der rechten Hälfte ein Gliederbereich **30** mit je zwei Paaren von Lochgliedern **31** definiert, die untereinander wieder durch Übergangsstege **33** miteinander verbunden sind. Aussen befindet sich der Verbindungssteg **10**. Zwischen den Lochgliedern **31'** und dem Gliederbereich **30** erstreckt sich der Führungsschlitz **40**, an den sich nach rechts und links die Spalte **41,41**' anschliessen, welche eng sind, so dass sich keine Erweiterung des Führungsschlitzes **40** ergibt. Flankiert wird der Führungsschlitz **40** von den parallel zur Längsachse **A** verlaufenden Plattenstreben **20**, die den Gliederbereich **30** mit den vorgelagerten Lochgliedern **31'** verbinden. Die Plattenstreben **20** weisen eine geringere Dicke auf als die übrigen Teile der Knochenplatte **1**. Dies begünstigt ein an die jeweils spezifische Knochengeometrie anpassendes Verbiegen der Knochenplatte **1** aus der flachen Ebene der Knochenplatte **1** heraus. In der Ebene - quasi über die Kante - ist eine höhere Steifheit gegeben. Die grössere Materialstärke ausserhalb der Plattenstreben **20** gibt den in die Schraubenlöcher **12,32** eingeschraubten Knochenschrauben **2** einen solideren Sitz. Der Feststeller **100** ist innerhalb des Führungsschlitzes **40** positioniert.

### Figuren 13A und 13B

Die Abwandlung zur vorherigen Figurengruppe 12A bis 12D besteht nur darin, dass jetzt der Gliederbereich **30** ein drittes Paar von Lochgliedern **31** aufweist und sowohl die Lochglieder **31** als auch die Schraubenlöcher **12** mit den vorgelagerten Lochgliedern **31'** einen grösseren Abstand zur Längsachse **A** haben, so dass verbreiterte Spalte **41,41'** entstehen, die eine beidseitige Verlängerung des Führungsschlitzes **40** ergeben. Der Feststeller **100** ist innerhalb des Spalts **41** positioniert und das äussere, nicht benötigte Paar von Lochgliedern **31** ist samt Verbindungssteg **10** und Übergangsstegen **33** abgeschnitten.

### Figuren 14A und 14B

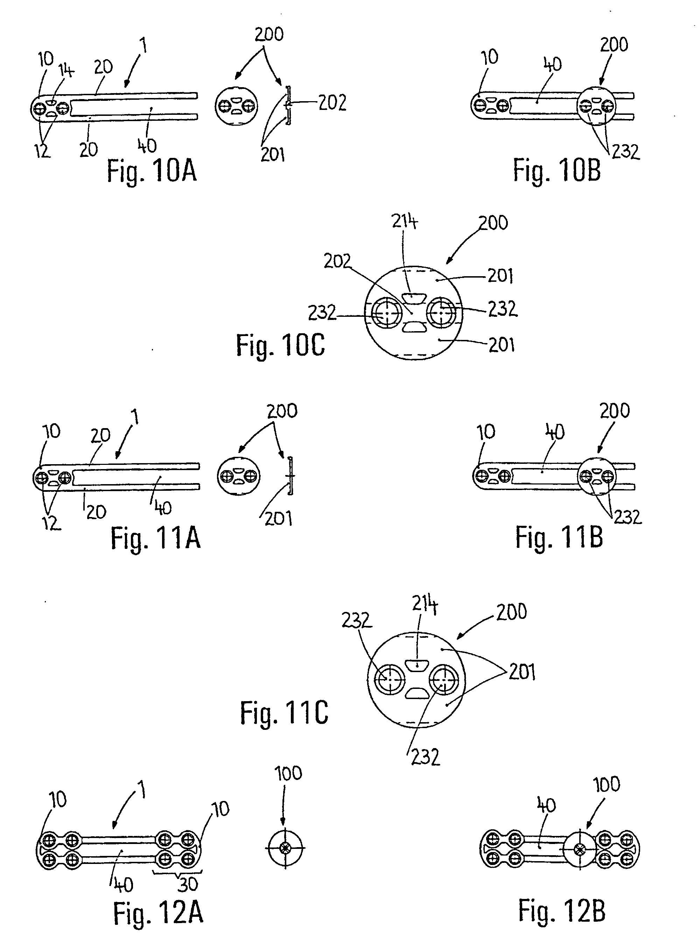

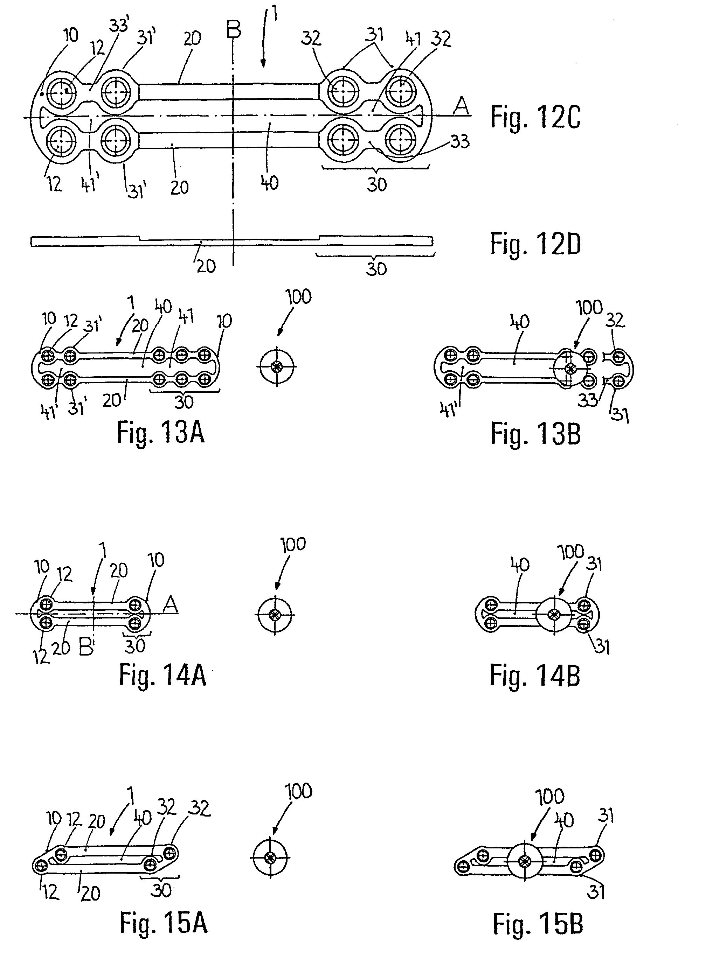

Die hier gezeigte Knochenplatte **1** ist im Verhältnis zur Figur 12A beidseitig um das Paar der vorgelagerten Lochglieder **31'** und ein Paar von Lochgliedern **31** aus dem Gliederbereich **30** reduziert. Der Führungsschlitz **40** - nur dort kann der Feststeller **100** folglich positioniert werden - erstreckt sich demnach nur zwischen dem Gliederbereich **30** und den Schraubenlöchern **12**. Mit Bezug auf die Längsachse **A** und die Vertikalachse **B** ist diese Knochenplatte **1** symmetrisch aufgebaut.

### Figuren 15A und 15B

In Abwandlung zu den Vorgängerfiguren 14A und 14B sind die Verbindungsstege **10** jetzt nicht mehr nierenförmig, sondern stellen die Schmalseiten einer insgesamt in Form eines Parallelogramms gestalteten Knochenplatte **1** dar. Die je zwei Schraubenlöcher **12,32** liegen damit nicht mehr auf zwei gemeinsamen theoretischen Vertikalen, jeweils das obere Schraubenloch **12,32** ist nach rechts versetzt. Der Führungsschlitz **40**, innerhalb dessen der Feststeller **100** eingesetzt ist, erstreckt sich wiederum zwischen dem Verbindungssteg **10** und dem Gliederbereich **30**.

### Figuren 16A, 16C und 16D

Gezeigt sind eine beidseits lateral geschlossene Knochenplatte **1** mit zwei äusseren horizontalen Verbindungsstegen **10** und ein seitlich fixierbaren Feststeller **100'**. Die Knochenplatte 1 ist bezüglich Längsachse **A** und Vertikalachse **B** symmetrisch konstruiert. Die Verbindungsstege **10** entsprechen der Ausführung gemäss Figur 1A. Auf die Gestaltung eines Führungsschlitzes **40** hat man hier verzichtet, da der Feststeller **100'** die Bohrung **111'** zum Einbringen der Feststellschraube **120** seitlich aufweist. Zur Erhöhung der Steifheit der Knochenplatte **1**, aber mit Beibehaltung der Biegbarkeit für den Zweck der Anpassung an die spezielle Knochengeometrie, sind zusätzlich zu den parallelen Plattenstreben **20** querverlaufende Verstärkungsstreben **21** vorgesehen. Die Verstärkungsstreben **21** erstrecken sich zwischen den beiden Verbindungsstegen **10**.

Der laschenförmige Feststeller **100'** ist zum Übergreifen der Knochenplatte **1** parallel zur Vertikalachse **B** konzipiert und besitzt an der unteren Peripherie die Bohrung **111'**. Die beidseits eingebrachten Einkerbungen **114'** sind komplementär zur Lage der Schraubenlöcher **12** angebracht, so dass der Feststeller **100'** nahe an einem Schraubenloch **12** und auch zwischen zwei Schraubenlöchern **12** stehen kann, ohne mit den einzudrehenden und durch die Schraubenköpfe erhöhten Knochenschrauben **2** in Kollision zu kommen. An der Unterseite weist der Feststeller **100'** oberhalb der Bohrung **111'** eine Nut **101'** in der Breite der Knochenplatte 1 auf, um letztere von der Oberseite partiell zu umfassen. Der Feststeller **100'** und die Feststellschraube **120** (s. Figur 1A) können zwei separate Teile sein oder eine voneinander nicht lösbare Einheit bilden. Als Feststellschraube **120** ist eine herkömmliche Knochenschraube **2** verwendbar.

### Figur 16B

Im aufgesetzten Zustand liegt die Bohrung **111'** des Feststellers **100'** seitlich, ausserhalb der Knochenplatte **1**, so dass die zugehörige Feststellschraube **120** in das darunter befindliche Knochenkompartiment - zunächst nicht angezogen-eingedreht werden kann. Ein noch lose aufgeschraubter Feststeller **100'** erlaubt wiederum die benötigten Realtivbewegungen für die Feinjustierung beider Knochenkompartimente zueinander. Bei der Feinjustierung ist der Feststeller **100'** um die Achse der Feststellschraube **120** schwenkbar und kann auf der Knochenplatte **1** entlang der Längsachse **A** verfahren werden. Nach der Feinjustierung erfolgt das vollständige Verschrauben der Knochenplatte **1** und hierauf das Entfernen des Feststellers **100'**.

### Figuren 17A und 17B

In dieser Variante ist - im Vergleich zu den Figuren 1D und 1E - die zentrische, duchgehende Bohrung **111** in der kreisrunden Scheibe **110** des Feststellers **100** nicht mit einem Innengewinde versehen. Um aber dennoch zu verhindern, dass die Scheibe **110** von der Feststellschraube **120** abfällt, ist die Bohrung **111** an der Scheibe **110** nur minim grösser als der Kerndurchmesser des Schraubenschafts **123** und jedenfalls kleiner als der Aussendurchmesser des Schraubenschafts **123.** Um dies zu erreichen, bieten sich Verstemmen oder Umbördeln einer zunächst grösseren Bohrung **111** an, damit zuvor der Schraubenschaft **123** durchgesteckt werden kann. Damit bleibt die in der Scheibe **110** steckende Feststellschraube **120** drehbar und die Scheibe **110** ist gegen Herunterfallen gesichert.

### Figuren 18A und 18B

In Abwandlung zur vorangehenden Ausführung besitzt die Scheibe **110** auf ihrer Unterseite am Aussenrand vier jeweils um 90° versetzte Arretiernasen **115**. Zwei gegenüber liegende Arretiernasen **115** umgreifen im aufgesetzten Zustand die Plattenstreben **20** einer unter dem Feststeller **100** positionierten Knochenplatte 1 parallel zur Vertikalachse **B**. Die zwei übrigen, auf der Längsachse **A** liegenden Arretiernasen **115**, dringen in den Führungsschlitz **40** ein.

### Figuren 19A und 19B

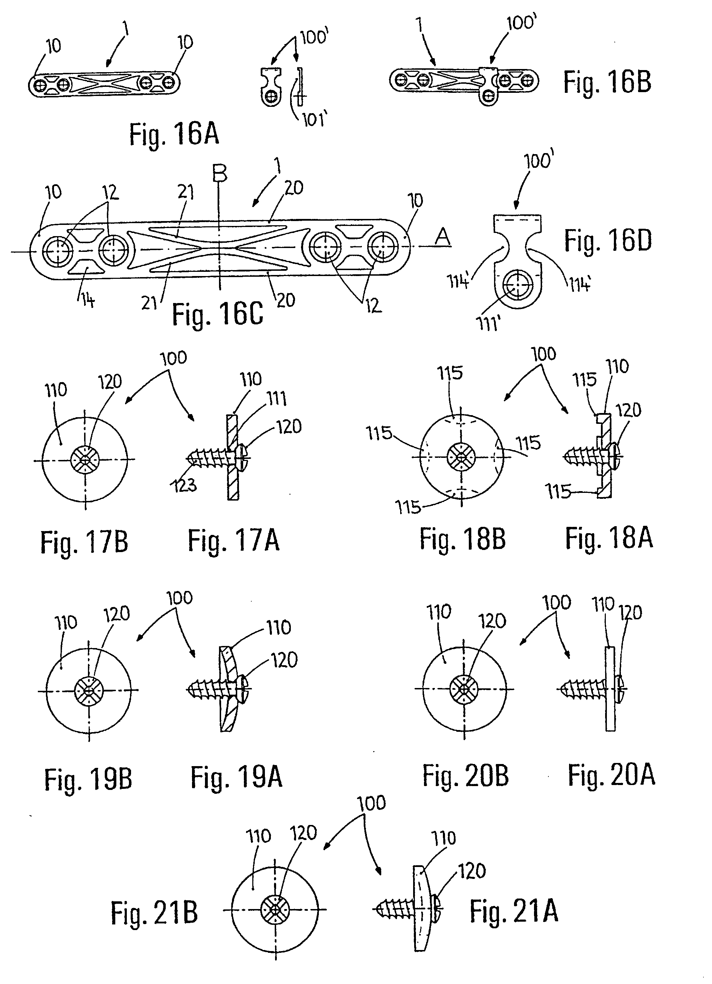

In Abwandlung zur Ausführung gemäss den Figuren 17A und 17B ist die Scheibe **110** gewölbt mit konkaver Kontur in Relation zur Spitze der in der Scheibe **110** steckenden Feststellschraube **120**. Die Wölbung ergibt im aufgeschraubten Zustand eine gewisse Federspannung, die für den besseren Andruck der Scheibe auf die darunter liegende Knochenplatte **1** vorteilhaft sein kann.

### Figuren 20A und 20B

Der Feststeller **100** könnte auch einteilig sein mit einer unter dem Schraubenkopf **122** sitzenden Scheibenteil **110**. Beim Drehen des Schraubenteils **120** dreht sich der Scheibenteil **110** dann stets mit.

### Figuren 21A und 21B

Nun weist der als einteilig ausgeführte Feststeller **100** ein gemäss den Figuren 19A und 19B gewölbtes Scheibenteil **110** auf.

## Patentansprüche

1. Vorrichtung zur osteosynthetischen Fixation von Knochenfragmenten, insbesondere von Fragmenten eines Kieferknochens, bestehend aus:
a) einer sich auf einer Längsachse (**A**) erstreckenden Knochenplatte (**1**) mit zwei zueinander beabstandeten, längslaufenden Plattenstreben (**20**), die zumindest am ersten Ende von einem Verbindungssteg (**10**) überbrückt sind;
aa) im Verbindungssteg (**10**) ist zumindest ein Schraubenloch (**12**) vorhanden;
ab) die Plattenstreben (**20**) flankieren entlang der Längsachse (A) beidseitig einen Zwischenraum, der einen Führungsschlitz (**40**) darstellt; und
b) einem zum temporären Aufsetzen auf die Knochenplatte (**1**), im Bereich des Führungsschlitzes (**40**), bestimmten Feststeller (**100**) mit einer Scheibe (**110**) und einer die Scheibe (110) fixierenden Feststellschraube (**120**);
ba) die Scheibe (**110**) übergreift den Führungsschlitz (**40**) und sitzt auf beiden Plattenstreben (**20**) auf;
bb) die Feststellschraube (**120**) durchdringt den Führungsschlitz (**40**) mit ihrem Schraubenschaft (**123**);
bc) der Schraubenkopf (**122**) sitzt auf der Scheibenoberseite (**113**) auf;
bd) der Führungsschlitz (**40**) besitzt eine der Längsachse (**A**) folgende Länge und eine quer zur Längsachse (**A**) sich erstreckende Breite, wobei die Länge ein Vielfaches der Breite beträgt und die Breite ein Vielfaches des Querschnitts des Schraubenschafts (**123**) beträgt, **dadurch gekennzeichnet, dass**
c) die Scheibe (**110**) und die Feststellschraube (**120**) eine voneinander nicht lösbare Einheit bilden.

2. Vorrichtung nach Anspruch 1, **dadurch gekennzeichnet, dass** der Feststeller (**100**) einstückig ist, wobei
a) die Feststellschraube (**120**) aus dem Schraubenkopf (**122**) und dem sich vom Schraubenkopf (**122**) axial erstreckenden Schraubenschaft (**123**) besteht; und
b) zwischen Schraubenkopf (**122**) und Schraubenschaft (**123**) ein horizontales Scheibenteil (**110**) angeordnet ist.

3. Vorrichtung nach Anspruch 1, **dadurch gekennzeichnet, dass** der Feststeller (100) zweiteilig ist, wobei
a) die Feststellschraube (**120**) aus dem Schraubenkopf (**122**) und dem sich vom Schraubenkopf (**122**) axial erstreckenden Schraubenschaft (**123**) besteht;
b) sich in der Scheibe (**110**) eine durchgehende Bohrung (**111**) befindet, durch welche der Schraubenschaft (**123**) der in Relation zur Scheibe (**110**) drehbaren Feststellschraube (**120**) ragt; und
c) die Scheibe (**110**) verliergesichert auf dem Schraubenschaft (**123**) angeordnet ist.

4. Vorrichtung nach einem der Ansprüche 1 bis 3, **dadurch gekennzeichnet, dass**
a) zuoberst im Schraubenkopf (**122**) eine Eingriffskontur (**124**) zum Ansetzen eines Schraubwerkzeugs vorhanden ist;
b) die Scheibe (**110**) an der dem Schraubenkopf (**122**) abgewandten Unterseite in Relation zur freien Spitze des Schraubenschafts (**123**) plan oder konkav ist; und
c) an der Unterseite der Scheibe (**110**) Arretiemasen (**115**) vorhanden sein können, welche die Plattenstreben (**20**) partiell umfassen.

5. Vorrichtung nach Anspruch 1, **dadurch gekennzeichnet, dass**
a) die Plattenstreben (**20**) am zweiten Ende der Knochenplatte (**1**), welches dem ersten Ende mit dem Verbindungssteg (**10**) gegenüber liegt, frei auslaufen; und somit
b) der Führungsschlitz (**40**) bis zum zweiten Ende hin ungeschmälert oder verengt offen ist.

6. Vorrichtung nach Anspruch 5, **dadurch gekennzeichnet, dass**
a) die Plattenstreben (**20**) am zweiten Ende jeweils einen Gliederbereich (**30**) aufweisen, wobei der Gliederbereich (**30**) altemativ besteht aus:
ba) gliedweise abtrennbaren Lochgliedern (**31**), die Schraubenlöcher (**32**) aufweisen; oder aus
bb) gliedweise abtrennbaren geraden Strebengliedern; oder aus
bc) gliedweise abtrennbaren Strebengliedern (**35**) mit systematisch sich wiederholenden Krümmungen.

7. Vorrichtung nach Anspruch 1, **dadurch gekennzeichnet, dass**
a) die den Führungsschlitz (**40**) beidseits längs flankierenden Plattenstreben (**20**) am zweiten Ende der Knochenplatte (**1**), welches dem ersten Ende mit dem Verbindungssteg (**10**) gegenüber liegt, ebenfalls von einem Verbindungssteg (**10**) überbrückt sind, in welchem Schraubenlöcher (**32**) vorhanden sein können; und somit
b) der Führungsschlitz (**40**) zu beiden Enden der Knochenplatte (**1**) hin geschlossen ist.

8. Vorrichtung nach Anspruch 1 oder 7, **dadurch gekennzeichnet, dass**
a) zwischen den Plattenstreben (20) und dem Verbindungssteg (10) am ersten Ende der Knochenplatte (**1**) und/oder dem Verbindungssteg (**10**) am zweiten Ende der Knochenplatte (**1**) zusätzliche Lochglieder (**31',31**) eingefügt sind; und
b) zumindest einer der Verbindungsstege (**10**) Durchbrüche (**14**) zum Eingriff eines an sich bekannten Halteinstruments aufweisen kann.

9. Vorrichtung nach Anspruch 1 und 8 oder 7 und 8, **dadurch gekennzeichnet, dass** der von den Plattenstreben (**20**) entlang der Längsachse (**A**) beidseits flankierte Führungsschlitz (**40**);
a) einerseits beginnt:
aa) am Verbindungssteg (**10**) am ersten Ende der Knochenplatte (1); oder
ab) an den diesem Verbindungssteg (**10**) vorgelagerten zusätzlichen Lochgliedem (**31'**); und
b) andererseits endet:
ba) vor den Gliederbereichen (**30**) an den Plattenstreben (**20**); oder
bb) am äusseren freien Ende der Gliederbereiche (**30**); oder
bc) vor dem Verbindungssteg (**10**) am zweiten Ende der Knochenplatte (**1**); oder
bd) vor den diesem Verbindungssteg (**10**) vorgelagerten zusätzlichen Lochgliedern (**31**).

10. Vorrichtung nach Anspruch 1, **dadurch gekennzeichnet, dass** für eine Knochenplatte (**1**), welche nur am ersten Ende einen Verbindungssteg (**10**) besitzt, eine Fixierplatte (**200**) mit zumindest einem Schraubenloch (**232**) zum Überbrücken der Plattenstreben (**20**) am zweiten Ende der Knochenplatte (**1**) vorgesehen ist.

11. Vorrichtung nach Anspruch 10, **dadurch gekennzeichnet, dass**
a) die Schraubenlöcher (**232**) der auf die Knochenplatte (**1**) aufgesetzten Fixierplatte (**200**):
aa) zwischen beiden Plattenstreben (**20**) oder den an den Plattenstreben (**20**) angesetzten Giederbereichen (**30**) zu liegen kommen; oder
ab) mit an den Giederbereichen (**30**) vorhandenen Schraubenlöchern (**32**) kongruent sind, so dass Knochenschrauben (**2**) in die übereinander liegenden Schraubenlöcher (**32,232**) einbringbar sind;
b) an der Unterseite der Fixierplatte (**200**) Konturen in Form von Vertiefungen (**201**) oder Begrenzungsstegen (**202**) vorgesehen sind, welche dem partiellen Einbetten von Gliedern (**31,35**) der Giederbereiche (**30**) dienen; und
c) die Fixierplatte (**200**) Durchbrüche (**14**) zum Eingriff eines an sich bekannten Halteinstruments aufweisen kann.

## Claims

1. A device for osteosynthetic fixation of bone fragments, in particular of fragments of a jaw bone, consisting of:
a) a bone plate (1) extending on a longitudinal axis (A) and with two spaced-apart and longitudinally extending plate struts (20) which are spanned by a connection web (10) at least at the first end;
aa) at least one screw hole (12) is present in the connection web (10);
ab) along the longitudinal axis (A), the plate struts (20) flank on both sides an interspace which constitutes a guide slot (40); and
b) a retainer (100) intended for temporary attachment to the bone plate (1), in the area of the guide slot (40), with a disk (110) and a locking screw (120) fixing the disk (110);
ba) the disk (110) engages over the guide slot (40) and sits on both plate struts (20);
bb) the locking screw (120) penetrates the guide slot (40) with its screw shank (123);
bc) the screw head (122) sits on the upper side (113) of the disk;
bd) the guide slot (40) has a length following the longitudinal axis (A) and a width extending transverse to the longitudinal axis (A), the length being a multiple of the width and the width being a multiple of the transverse section of the screw shank (123), **characterised in that**
c) the disk (110) and the locking screw (120) form an inseparable unit.

2. The device according to claim 1, **characterised in that** the retainer (100) is in one piece, wherein
a) the locking screw (120) consists of the screw head (122) and the screw shank (123) which extends axially from the screw head (122); and
b) a horizontal disk part (110) is arranged between screw head (122) and screw shank (123).

3. The device according to claim 1, **characterised in that** the retainer (100) is in two pieces, wherein
a) the locking screw (120) consists of the screw head (122) and the screw shank (123) which extends axially from the screw head (122);
b) in the disk (110) a through-bore (111) is situated, through which the screw shank (123) of the locking screw (120), which is rotatable in relation to the disk (110), penetrates; and
c) the disk (110) is arranged on the screw shank (123) in a manner secure against loss.

4. The device according to one of claims 1 to 3, **characterised in that**
a) at the top of the screw head (122) there is an engagement contour (124) for application of a screwing instrument;
b) on the underside facing away from the screw head (122), the disk (110) is plane or concave in relation to the free point of the screw shank (123); and
c) catches (115) can be present on the underside of the disk (110) and partially enclose the plate struts (20).

5. The device according to claim 1, **characterised in that**
a) the plate struts (20) run out freely at the second end of the bone plate (1) lying remote from the first end with the connection web (10); and thus
b) the guide slot (40) is open as far as the second end, with or without narrowing.

6. The device as claimed in claim 5, **characterised in that**
a) the plate struts (20) have an area of members (30) in each case at the second end, said area of members (30) alternatively consisting of:
ba) holed members (31) which can be divided off per member and which have screw holes (32); or of
bb) straight strut members which can be divided off per member; or of
bc) strut members (35) which can be divided off by member and have systematically repeating curves.

7. The device according to claim 1, **characterised in that**
a) the plate struts (20) flanking the length of the guide slot (40) on both sides are likewise spanned by a connection web (10) which is at the second end of the bone plate (1), remote from the first end with the connection web (10), and in which screw holes (32) can be present; and thus
b) the guide slot (40) is closed at both ends of the bone plate (1).

8. The device according to claim 1 or 7, **characterised in that**
a) additional holed members (31'; 31) are inserted between the plate struts (20) and the connection web (10) at the first end of the bone plate (1) and/or the connection web (10) at the second end of the bone plate (1); and
b) at least one of the connection webs (10) has apertures (14) for engagement of a holder instrument known per se.

9. The device according to claims 1 and 8 or 7 and 8, **characterised in that** the guide slot (40) flanked on both sides by the plate struts (20) along the longitudinal axis (A):
a) on one hand begins:
aa) on the connection web (10) at the first end of the bone plate (1); or
ab) at the additional holed members (31') mounted forward of this connection web (10); and
b) on the other hand terminates;
ba) before the areas of members (30) on the plate struts (20); or
bb) on the outer free end of the area of members (30); or
bc) before the connection web (10) at the second end of the bone plate (1); or
bd) before the additional holed members (31) mounted forward of this connection web (10).

10. The device according to claim 1, **characterised in that** for a bone plate (1) which has a connection web (10) only at the first end, a fixing plate (200) with at least one screw hole (232) is provided for spanning the plate struts (20) at the second end of the bone plate (1).

11. The device according to claim 10, **characterised in that**
a) the screw holes (232) of the fixing plate (200) attached to the bone plate (1):
aa) come to lie between both plate struts (20) or the areas of members (30) mounted on the plate struts (20); or
ab) are congruent with screw holes (32) present on the areas of members (30), so that bone screws (2) can be introduced into the screw holes (32; 232) lying one above the other;
b) on the underside of the fixing plate (200), contours in the form of depressions (201) or boundary webs (202) are provided which are used for partially embedding members (31, 35) of the areas of members (30); and
c) the fixing plate (200) can have apertures (14) for engagement of a holder instrument known per se.

## Revendications

1. Dispositif pour la fixation ostéosynthétique de fragments d'os, notamment de fragments d'un os de mâchoire, constitué de :
a) une plaque à os (1) s'étendant sur un axe longitudinal (A), pourvue de deux tirants (20) de plaque espacés, longitudinaux, qui au moins à la première extrémité sont pontés par une patte de liaison (10) ;
aa) dans la patte de liaison (10) il est prévu au moins un trou de vis (12) ;
ab) de chaque côté le long de l'axe longitudinal (A) les tirants de plaque (20) flanquent un espace intercalaire qui constitue une fente de guidage (40) ; et
b) un fixateur (100) destiné à la mise en place temporaire sur la plaque à os (1), dans la zone de la fente de guidage (40), comportant un disque (110) et une vis de fixation (120) fixant le disque (110) ;
ba) le disque (110) recouvre la fente de guidage (40) et s'appuie sur les deux tirants de plaque (20);
bb) la vis de fixation (120) traverse la fente de guidage (40) par sa tige filetée (123);
bc) la tête de vis (122) s'appuie sur le côté supérieur (113) du disque ;
bd) la fente de guidage (40) présente une longueur suivant l'axe longitudinal (A) et une largeur s'étendant transversalement à l'axe longitudinal (A), la longueur formant un multiple de la largeur et la largeur un multiple de la section transversale de la tige filetée (123), **caractérisé en ce que**
c) le disque (110) et la vis de fixation (120) constituent une unité non séparable.

2. Dispositif selon la revendication 1, **caractérisé en ce que** le fixateur (100) est d'une seule pièce, dans laquelle
a) la vis de fixation (120) est constituée de la tête de vis (122) et de la tige filetée (123) s'étendant axialement à partir de la tête de vis (122) ; et
b) une pièce de disque horizontale (110) est disposée entre la tête de vis (122) et la tige filetée (123).

3. Dispositif selon la revendication 1, **caractérisé en ce que** le fixateur (100) est en deux pièces, dans lequel
a) la vis de fixation (120) est constituée de la tête de vis (122) et de la tige filetée (123) s'étendant axialement à partir de la tête de vis (122) ;
b) un perçage traversant (111) est agencé dans le disque (110), qui est traversé par la tige filetée (123) de la vis de fixation (120), rotative par rapport au disque (110) ; et
c) le disque (110) est disposé de façon imperdable sur la tige filetée (123).

4. Dispositif selon l'une des revendications 1 à 3, **caractérisé en ce que**
a) tout en haut dans la tête de vis (122) il est prévu un profil de préhension (124) pour la mise en place d'un outil de vissage ;
b) le côté de dessous du disque (110) tourné à l'opposé de la tête de vis (122) par rapport à la pointe libre de la tige filetée (123) est plan ou concave ; et
c) des nez d'arrêt (115) peuvent être prévues sur le côté de dessous du disque (110), lesquelles entourent partiellement les tirants de plaque (20).

5. Dispositif selon la revendication 1, **caractérisé en ce que**
a) les tirants de plaque (20) se terminent librement sur la deuxième extrémité de la plaque à os (1), extrémité qui est située à l'opposé de la première extrémité avec la patte de liaison (10) ; et de ce fait
b) la fente de guidage (40) est ouverte jusqu'à la deuxième extrémité sans être réduite ou rétrécie.

6. Dispositif selon la revendication 5, **caractérisé en ce que**
a) les tirants de plaque (20) comportent respectivement une zone d'organes (30) à la seconde extrémité, la zone d'organes (30) étant constituée en alternativement :
ba) d'organes à trous (31) séparables par organe, qui comportent des trous de vis (32) ; ou
bb) d'organes de tirants droits, séparables par organe ; ou
bc) d'organes de tirants (35) séparables par organe, agencés avec des courbures se répétant systématiquement.

7. Dispositif selon la revendication 1, **caractérisé en ce que**
a) sur la deuxième extrémité de la plaque à os (1) qui se trouve à l'opposé de la première extrémité avec la patte de liaison (10), les tirants de plaque (20) qui flanquent longitudinalement des deux côtés la fente de guidage (40) sont également pontés par une patte de liaison (10), dans laquelle des trous de vis (32) peuvent être prévus ; et de ce fait
b) la fente de guidage (40) est fermée aux deux extrémités de la plaque à os (1).

8. Dispositif selon l'une des revendications 1 ou 7, **caractérisé en ce que**
a) des organes à trous (31', 31) supplémentaires sont insérés entre les tirants de plaque (20) et la patte de liaison (10) à la première extrémité de la plaque à os (1), et/ou sur la patte de liaison (10) à la seconde extrémité de la plaque à os (1) ; et
b) au moins l'une des pattes de liaison (10) peut comporter des ouvertures de passage (14) pour la préhension d'un instrument de maintien connu en soi.

9. Dispositif selon les revendications 1 et 8 ou 7 et 8, **caractérisé en ce que** la fente de guidage (40) flanquée des deux côtés par les tirants de plaque (20) le long de l'axe longitudinal (A) ;
a) d'une part commence:
aa) à la patte de liaison (10) à la première extrémité de la plaque à os (1) ; ou
ab) aux organes à trous supplémentaires (31') disposés devant cette patte de liaison (10) ; et
b) d'autre part se termine :
ba) avant les zones d'organes (30) sur les tirants de plaque (20) ; ou
bb) à l'extrémité libre extérieure de la zone d'organes (30) ; ou
bc) avant la patte de liaison (10) à la seconde extrémité de la plaque à os (1) ; ou
bd) avant les organes à trous supplémentaires (31) disposés devant cette patte de liaison (10).

10. Dispositif selon la revendication 1, **caractérisé en ce qu'**il est prévu, à la deuxième extrémité de la plaque à os (1), une plaque de fixation (200) pourvue d'au moins un trou de vis (232) pour ponter les tirants de plaque (20), pour une plaque à os (1) qui possède une plaque de liaison (10) seulement à la première extrémité.

11. Dispositif selon la revendication 10, **caractérisé en ce que**
a) les trous de vis (232) de la plaque de fixation (200) mise en place sur la plaque à os (1):
aa) se placent entre les deux tirants de plaque (20) ou les zones d'organes (30) aménagées sur les tirants de plaque (20) ; ou
ab) sont congruents avec les trous de vis (32) prévus dans les zones d'organes (30), de sorte que les vis à os (2) peuvent être introduites dans les trous de vis superposés (32,232);
b) des profils sont prévus sur la face inférieure de la plaque de fixation (200), sous la forme d'empreintes (201) ou de pattes de délimitation (202), qui servent à noyer partiellement des organes (31,35) des zones d'organes (30) ; et
c) la plaque de fixation (200) peut comporter des ouvertures de passage (14) pour la préhension d'un instrument de maintien connu en soi.
